# EUROPÄISCHE PATENTSCHRIFT

(11) **EP 1 516 182 B1**
(45) Veröffentlichungstag und Bekanntmachung des Hinweises auf die Patenterteilung: **25.08.2010**
(21) Anmeldenummer: 03735519.5
(22) Anmeldetag: 02.06.2003
(51) Int. Cl.: G01N 33/50, G01N 33/574

(54) **VERFAHREN ZUR EIGENSCHAFTSBESTIMMUNG UND/ODER KLASSIFIKATION VON ZIRKULIERENDEN MACROPHAGEN SOWIE ANALYSEANORDNUNG ZUR DURCHFÜHRUNG DES VERFAHRENS**
METHOD FOR THE DETERMINATION OF CHARACTERISTICS AND/OR THE CLASSIFICATION OF CIRCULATING MACROPHAGES, AND ANALYSIS ARRANGEMENT FOR CARRYING OUT SAID METHOD
PROCEDE PERMETTANT LA DETERMINATION DE PROPRIETES ET/OU LA CLASSIFICATION DE MACROPHAGES CIRCULANTS ET ENSEMBLE D'ANALYSE SERVANT A LA MISE EN OEUVRE DUDIT PROCEDE

(30) Priorität: 26.06.2002 DE 10228548; 09.07.2002 DE 10230893
(43) Veröffentlichungstag der Anmeldung: 23.03.2005
(73) Patentinhaber: Herwig, Ralf, 6363 Westendorf (AT)
(72) Erfinder: Herwig, Ralf, 6363 Westendorf (AT)
(74) Vertreter: Müllner, Martin
(86) Internationale Anmeldenummer: PCT/EP2003/005763
(87) Internationale Veröffentlichungsnummer: WO 2004/003548

(56) Entgegenhaltungen:
- DE-A- 19 850 049
- HERWIG RALF ET AL: "Circulating blood macrophages of prostate cancer patients contain PSA in combination with structures of epthelial origin." JOURNAL OF UROLOGY, Bd. 169, Nr. 4 Supplement, April 2003 (2003-04), Seite 52 XP009019758 98th Annual Meeting of the American Urological Association (AUA);Chicago, IL, USA; April 26-May 02, 2003 ISSN: 0022-5347
- DE CAESTECKER M P ET AL: "THE DETECTION OF INTRACYTOPLASMIC INTERLEUKIN-1-ALPHA INTERLEUKIN-1-BETA AND TUMOUR NECROSIS FACTOR ALPHA EXPRESSION IN HUMAN MONOCYTES USING TWO COLOUR IMMUNOFLUORESCENCE FLOW CYTOMETRY" JOURNAL OF IMMUNOLOGICAL METHODS, Bd. 154, Nr. 1, 1992, Seiten 11-20, XP009019859 ISSN: 0022-1759
- BRANDT BURKHARD ET AL: "Isolation of prostate-derived single cells and cell clusters from human peripheral blood" CANCER RESEARCH, Bd. 56, Nr. 20, 1996, Seiten 4556-4561, XP001155823 ISSN: 0008-5472 in der Anmeldung erwähnt
- KUSABA MIKAKO ET AL: "Analysis of type 1 and type 2 T cells in synovial fluid and peripheral blood of patients with rheumatoid arthritis" JOURNAL OF RHEUMATOLOGY, Bd. 25, Nr. 8, August 1998 (1998-08), Seiten 1466-1471, XP009019858 ISSN: 0315-162X
- SINHA A A ET AL: "IMMUNOELECTRON MICROSCOPIC LOCALIZATION OF PROSTATIC-SPECIFIC ANTIGEN IN HUMAN PROSTATE BY THE PROTEIN A-GOLD COMPLEX" CANCER, Bd. 60, Nr. 6, 1987, Seiten 1288-1293, XP001155947 ISSN: 0008-543X in der Anmeldung erwähnt
- HERWIG R; HORNINGER W; REHDER P; KLOCKER H; RAMONER R; THURNHER M; PINGGERA G M; GOZZI C; KONWALINKA G; BARTSCH G: "Ability of PSA-positive circulating macrophages to detect prostate cancer" PROSTATE 20050215 US, vol. 62, no. 3, 15 February 2005 (2005-02-15), pages 290-298, ISSN: 0270-4137
- HERWIG R; MITTEREGGER D; DJAVAN B; KRAMER G; MARGREITER M; LEERS M P; GLODNY B; HAIDER D G; HORL W H; MARBERGER M: "Detecting prostate cancer by intracellular macrophage prostate-specific antigen (PSA): A more specific and sensitive marker than conventional serum total PSA" EUROPEAN JOURNAL OF CLINICAL INVESTIGATION 200806 GB, vol. 38, no. 6, June 2008 (2008-06), pages 430-437, ISSN: 0014-2972 1365-2362
- HERWIG R; PELZER A; HORNINGER W; REHDER P; KLOCKER H; RAMONER R; PINGGERA G M; GOZZI C; KONWALINKA G; BARTSCH G: "Measurement of intracellular versus extracellular prostate-specific antigen levels in peripheral macrophages: A new approach to noninvasive diagnosis of prostate cancer" CLINICAL PROSTATE CANCER 200412 US, vol. 3, no. 3, December 2004 (2004-12), pages 184-188, ISSN: 1540-0352

## Beschreibung

Die Erfindung betrifft ein Verfahren zur Eigenschaftsbestimmung und/oder Klassifikation von zirkulierenden Macrophagen durch nicht zelleigene Antigene sowie eine Analyseanordnung zur Durchführung eines solchen Verfahrens.

Aus Sinha, Wilson, Gleason: Immunoelectron microscopic localization of prostaticspecific antigen in human prostate by the protein A-gold complex, Cancer, 1987, 60, 1288-91, ist es bekannt, elektronenmikroskopische Untersuchungen von Zellen aus Prostatageweben vorzunehmen, wobei nach der erwähnten Literaturstelle normale Gewebe aus der Prostata, Prostata-Karzinomgewebe und Prostata-Hyperplasiegewebe mit Gold-gelabelten PSA-Antikörpern inkubiert wurden. Die Untersuchungen ergaben, dass sich die Goldpartikel im Cytoplasma, in intrazellulären Granula, dem RES und Lysosomen befinden. Mit zunehmender Entdifferenzierung eines Tumors erscheinen mehr Gold-Partikel in Membranstrukturen. Dies wurde als ein Zeichen dafür bewertet, dass PSA (Prostata-spezifisches-Antigen) mit zunehmender Entdifferenzierung der Tumorzellen in Membranstrukturen eingelagert wird. Ein weiterer Aspekt der dortigen Untersuchung war, dass Gold-Partikel ebenfalls in Granulozyten und Macrophagen erkannt wurden.

Bei durchflusszytometrischen Untersuchungen wurden Zellen im zirkulierenden Blut gefunden, die PSA-positiv waren. Bei den vorbekannten Untersuchungen wurden aber nur die Oberflächen der Macrophagen für PSA gefärbt.

Die Tatsache, dass keine mRNA des PSA-Moleküls in den Macrophagen gefunden wurde, lässt nur den Schluss zu, dass nur das PSA-Molekül aufgenommen wird und es sich nicht um die Elimination von Mikrometastasen handelt. Verwiesen sei hier auf Brandt, Griwatz, Brinkmann: Circulating prostate-specific antigen/CD14-double-positive cells; a blomarker indicating low risk for hematogeneous metastasis of prostate cancer, J.Natl. Cancer Inst. 1997; 89, 174.

Bekanntermaßen werden bösartige Veränderungen von gewebsständigen Zellen, die in einem mehr oder weniger geordneten Zellhaufen zusammenliegen, als Tumor bezeichnet. Diese Tumorzellen missachten die Ordnung der Gewebe, wachsen ungehemmt und expandieren durch Größenzunahme sowie durch Infiltration in das andere umliegende Gewebe, Organ, oder wachsen über die Organgrenzen hinaus in die Blutbahn und das Lymphsystem.
Erreicht ein Tumor die Blutbahn oder das Lymphsystem, so können hier die Einzelzellen oder Zellhaufen über diese Systeme abschwimmen und sich als Tochtergeschwülste, d.h. Metastasen an anderen Orten des Körpers festsetzen. Hier besteht die Gefahr, dass die Metastasen weiter wachsen und dem Körper Energie rauben, bis dieser schließlich verfällt und seiner Erkrankung erliegt.
Bei der Entwicklung eines solchen Tumors werden von den Tumorzellen Substanzen produziert, die dazu dienen, dieses Wachstum zu unterstützen. Zusätzlich können Stoffe freigesetzt werden, die als Indikator für Tumorwachstum Verwendung finden. Letztere werden als Tumormarker bezeichnet. Diese Marker sind aber nicht spezifisch für einen Tumor, sondern nur die Höhe der gemessenen Konzentration im Blut, da auch gesunde Zellen derartige Stoffe freisetzen können. Tumormarker können daher nicht der Auffindung eines Tumors, sondern nur zur Kontrolle des Krankheits- oder Therapieverlaufs herangezogen werden. Ein spezifischer Marker für einen Tumor ist das Prostata-spezifische-Antigen (PSA), welches ab einer gewissen Konzentration im Blut auf ein Prostatakarzinom hinweist. Eine gutartige Vergrößerung der Prostata kann allerdings auch eine Erhöhung des PSA-Werts im Blut hervorrufen.

Bisher werden Tumorerkrankungen vorwiegend über bildgebende Verfahren, wie Ultraschall oder Computertomographie, Mammographie oder dergleichen diagnostiziert. Eine endgültige Entscheidung erfolgt allerdings erst nach einer tumorhaltigen Gewebeprobe mit Festlegung des Therapieverlaufs.

Das dem menschlichen Körper eigene Immunsystem wirkt Tumorerkrankungen entgegen. Dieses Immunsystem besteht aus einer Reihe von verschiedenen Zelltypen, die unterschiedliche Aufgaben erfüllen. Unter anderem haben die Macrophagen die Aufgabe, bestimmtes krankhaftes Material zu erkennen und es zu phagozytieren und in seine Bestandteile zu zerlegen. Anschließend werden Fragmente der gefressenen Zellen an der Oberfläche anderen Immunzellen präsentiert, damit diese die Möglichkeit haben, die Struktur zu erkennen, gegen die vorgegangen werden soll.

Es besteht ein dringendes Bedürfnis, bereits in einem frühzeitigen Stadium eine Eigenschaftsbestimmung von zirkulierenden Macrophagen vorzunehmen, ohne dass unmittelbar Untersuchungen am menschlichen Körper vorgenommen werden müssen.

Die Aufgabe der Erfindung besteht darin, ein Verfahren sowie eine Analyseanordnung anzugeben, mit deren Hilfe eine Eigenschaftsbestimmung und/oder Klassifikation von zirkulierenden Macrophagen möglich wird.

Erfindungsgemäß wird davon ausgegangen, dass Antigene oder Fragmente von phagozytierten Tumorzellen in zirkulierenden Macrophagen nachweisbar sind, so dass damit ein direkter und spezifischer Tumornachweis geführt werden kann.

Erfindungsgemäß wird eine Entnahme von Vollblut mit anschließender Gradientenzentrifugation zur Isolierung von Macrophagen vorgenommen. Die Macrophagenzellen werden dann perforiert und es wird eine intrazelluläre Färbung der Zellen mit mindestens einem ausgewählten Antikörper realisiert.

Im Anschluss wird auf die an sich bekannte Durchflusszytometrie zurückgegriffen, um die Zelleigenschaften auf Einzelebene zu dokumentieren.

Die Durchflusszytometrie ermöglicht das Zählen und die Analyse von physikalischen und molekularen Eigenschaften von Zellen in einem Flüssigkeitsstrom. Konkret wird mit Hilfe von Fluoreszenzfarbstoff-marklerten Proben, z.B. Antikörpern, eine Bestimmung der Eigenschaften von Zellen oder Zellpopulationen auf Einzelebene vorgenommen und dokumentiert.

Grundlage ist hier die Antigen-Antikörper-Reaktion, die mit Fluoressenzfarbstoff markierten Antikörpern durchgeführt wird. Zur Analyse werden die Zellen einer Einzelsuspension durch hydrodynamische Fokussierung an einem gebündelten Laserstrahl geeigneter Wellenlänge vorbeigeleitet. Bei entsprechender Anregung der Elektronen des Fluoreszenzfarbstoffs durch den monochromatischen Laserstrahl werden diese auf ein höheres Energieniveau gehoben. Nach dem Laserpuls fallen die Elektronen unter Abgabe von Energie in Form von Photonen auf ihr Ursprungsniveau zurück. Die emittierte Photonenkonzentration, die durch einen Photodetektor registriert wird, verhält sich proportional zur Menge an gebundenen Antikörpern je Zelle. Zusätzlich werden durch die Lichtbeugung und -streuung Informationen über die Zellgröße und die Binnenstruktur, d.h. Granularität des Zytoplasmas, die Größe des Zellkerns und so weiter gewonnen.

Als ausgewählte Antikörper kommen Prostata-spezifische Antigene, Cytokeratin-Antikörper und/oder epitheliales Membranantigen zur Anwendung.

Über die Färbung des PSA-Antikörpers in den Macrophagen ist dann erfindungsgemäß bestimmbar, ob das phagozytierte Material prostatarelevant ist.

Die Analyseanordnung zur Durchführung des Verfahrens umfasst Mittel zur Heparinisierung von entnommenem Blut, einen Grandientenzentrifugator zur Isolation von Macrophagen, Mittel zur Zellperforation, eine Einrichtung zur intrazellulären Färbung mit fluorochromierten Antikörpern der vorbehandelten Zellen und ein Durchflusszytometer mit rechnergestützter Auswerteeinheit zum Feststellen der intrazellulären Struktur der jeweils isolierten und vorbehandelten Zelle zum Zwecke der Tumorfrüherkennung.

Die Erfindung soll nachstehend anhand eines Ausführungsbeispiels näher erläutert werden.

Im Schritt der Blutentnahme und Färbung wird von beispielsweise 6 ml Vollblut ausgegangen, das einer Heparinisierung unterzogen wird. Mit Hilfe der Grandientenzentrifugation erfolgt eine Isolierung von Monozyten, Macrophagen und Lymphozyten.

In einem nächsten Schritt wird eine Formaldehydfixierung und Saponinbehandlung der Zellen zum Zwecke der Perforation durchgeführt.

Folgend schließt sich der Schritt der intrazellulären Färbung mit ausgewählten Antikörpern z.B. der nachstehenden Tabelle an.
PSA-Antikörper Ab-1 (Clone ER-PRS)
Pan-Cytokerati n-FITC
Epithelial Membrane Antigen (Clone E 29)
Isotypenkontrolle IgG1 (Clone DAK-GO1)
Sekundärantikörper FITC Ziege-Anti-Maus (DAKO)

Die entsprechend bis zur Analyse erneut fixierte Zelle wird dann mittels Durchflusszytometrie näher untersucht. Monozyten und Macrophagen werden gegated, d.h. es wird nur ein Teil der Messergebnisse zur Auswertung herangezogen und eine Vorauswahl getroffen.

Über eine Histogrammauswertung wird dann die Isotypenkontrolle und Färbung beurteilt und es erfolgt eine Angabe der positiven Zellen, z.B. in Prozent.

Es hat sich gezeigt, dass bei der Färbung der Macrophagen mit Cytokeratin bei Patienten mit verstreutem Prostatatumor Anteile der Baustruktur von Gewebezellen in den zirkulierenden Immunzellen der entsprechenden Person zu finden sind. Da diese Bauelemente nicht ursprüngliche Inhalte der Immunzellen darstellen, müssen diese durch Phagozytose aufgenommen worden sein. Ein unspezifischer Effekt kann ausgeschlossen werden, da der aufgenommene Kurvenverlauf des Cytokeratins sich deutlich von dem des Isotyps unterscheidet.

Die Färbung des PSA in Macrophagen beweist, dass es sich bei dem phagozytierten Material um Prostatagewebe handeln muss, da auch dieser spezifische Marker nachweisbar ist.

Insgesamt ist mit dem beschriebenen Verfahren und der zugehörigen Analyseanordnung eine neuartige Methode zur Eigenschaftsbestimmung und Klassifikation von zirkulierenden Macrophagen gegeben, wobei die Klassifikation Aussagen über mögliche prostatarelevante Inhalte zulässt.

## Patentansprüche

1. Verfahren zur Eigenschaftsbetimmung und/oder Klassifikation von zirkulierenden Macrophagen mit folgenden Schritten:
- Isolierung von Macrophagen aus Vollblut mittels Gradientenzentrifugation,
- Perforation der Macrophagenzellen,
- intrazelluläre Färbung der Zellen mit mindestens einem ausgewählten Antikörper sowie
- durchflußzytometrische Analyse der vorbehandelten Zellen mit anschließender statistischer Bewertung über mehrere Zellen.

2. Verfahren nach Anspruch 1,
**gekennzeichnet durch**
die Verwendung von Prostata-spezifischen Antigen (PSA), Cytokeratin und/oder epitheliales Membranantigen als dem oder den auszuwählenden Antikörpern.

3. Verfahren nach Anspruch 1 oder 2,
**gekennzeichnet durch**
Histogrammauswertung der Isotypenkontrolle und Färbung nach durchgeführter Durchflußzytometrie.

4. Verfahren nach einem der vorangegangenen Ansprüche zum Erkennen von durch Phagozytose aufgenommenen Anteilen von Gewebezellen eines verstreuten Prostatatumors außerhalb des menschlichen Körpers.

5. Verfahren nach Anspruch 4,
**dadurch gekennzeichnet, daß**
über die Färbung des PSA in den Macrophagen bestimmt wird, ob das phagozytierte Material prostatarelevant ist.

## Claims

1. Method for determining properties of and/or classifying circulating macrophages, comprising the following steps:
- isolating macrophages from whole blood by gradient centrifugation,
- perforating the macrophage cells,
- intracellular staining of the cells with at least one selected antibody, and
- flow cytometric analysis of the pre-treated cells, with subsequent statistical evaluation of a plurality of cells.

2. Method according to claim 1, **characterised by** the use of prostate-specific antigen (PSA), cytokeratin and/or epithelial membrane antigen as said selected antibody or antibodies.

3. Method according to either claim 1 or claim 2, **characterised by** histogram analysis of the isotype control and staining after flow cytometry has been carried out.

4. Method according to any one of the preceding claims for detecting portions of tissue cells of a scattered prostate tumour, which portions are taken up by phagocytosis, outside the human body.

5. Method according to claim 4, **characterised in that** it is determined from the staining of the PSA in the macrophages whether the material taken up by phagocytosis is prostate-relevant.

## Revendications

1. Procédé de détermination des propriétés et/ou de classification de macrophages en circulation, lequel procédé comporte les étapes suivantes :
- l'isolation des macrophages provenant du sang total au moyen de la centrifugation sur gradient,
- la perforation des cellules macrophages,
- la coloration intracellulaire des cellules avec au moins un anticorps sélectionné ainsi que
- l'analyse par cytométrie en flux des cellules prétraitées avec évaluation statistique subséquente de plusieurs cellules.

2. Procédé selon la revendication 1, **caractérisé par** l'utilisation d'antigènes spécifiques à la prostate (PSA), de cytokératine et/ou d'antigènes membranaires épithéliaux en tant qu'anticorps à sélectionner.

3. Procédé selon la revendication 1 ou 2, **caractérisé par** l'analyse de l'histogramme du contrôle isotypique et la coloration après la réalisation de la cytométrie en flux.

4. Procédé selon l'une quelconque des revendications précédentes pour la reconnaissance, à l'extérieur du corps humain, de parties de cellules tissulaires, ingérées par phagocytose, d'une tumeur de la prostate disséminée.

5. Procédé selon la revendication 4, **caractérisé en ce qu'**on détermine, au moyen de la coloration du PSA dans les macrophages, si le matériel phagocyté concerne ou pas la prostate.
